(19) Europäisches Patentamt
European Patent Office
Office européen des brevets

(11) Publication number: 0 181 128 B1

(12) EUROPEAN PATENT SPECIFICATION

(45) Date of publication of patent specification: 04.10.89

(51) Int. Cl.[4]: C 07 H 19/167, A 61 K 31/70

(21) Application number: 85307715.4

(22) Date of filing: 25.10.85

# (54) N6-bicycloadenosines.

(30) Priority: 26.10.84 US 665216
09.09.85 US 772983

(43) Date of publication of application:
14.05.86 Bulletin 86/20

(45) Publication of the grant of the patent:
04.10.89 Bulletin 89/40

(84) Designated Contracting States:
AT BE CH DE FR GB IT LI LU NL SE

(56) References cited:
EP-A-0 040 325
DE-A-2 249 397
US-A-3 590 029
US-A-3 838 147
US-A-3 840 521

JOURNAL OF MEDICINAL CHEMISTRY, vol. 28, no. 11, 1985, pages 1636-1643, American Chemical Society, Columbus, Ohio, US; S. KUSACHI et al.: "Dog coronary artery adenosine receptor: Structure of the N6-alkyl subregion"

(73) Proprietor: WARNER-LAMBERT COMPANY
201 Tabor Road
Morris Plains New Jersey 07950 (US)

(72) Inventor: Trivedi, Bharat
44833 Tillotson Drive
Canton Michigan 48187 (US)

(74) Representative: Jones, Michael Raymond et al
HASELTINE LAKE & CO. Hazlitt House 28
Southampton Buildings Chancery Lane
London WC2A 1AT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 181 128 B1

**Description**

Netherlands Patent 2,249,396 and European Patent Publication 40325 disclose $N^6$-bicycloalkyl adenosine derivatives having circulatory, antilipolytic, anti-convulsant, muscle relaxant, and sedative activity. US Patent 3,840,521 discloses $N^6$-methyl-$N^6$-[bicyclo(2.2.1)-heptyl-2]adenosine having antilipolytic, anti-hyperlipemic, and antihypercholesterolemic activity. $N^6$-bicycloalkyl adenosine derivatives having similar properties are also disclosed in US Patent 3,838,147 and German Offenlegungsschrift 2,249,397.

The compounds of the instant invention are adenosine analogs having some of the same activity as adenosine, but having a significantly longer duration of action. A distinguishing feature of these compounds from other adenosine analogs previously described, is the discovery that $N^6$-bicycloadenosines have favorable ratio of affinities at A1 and A2 receptors and highly desirable analgesic and antiinflammatory properties.

Accordingly the present invention relates to compound of the formula

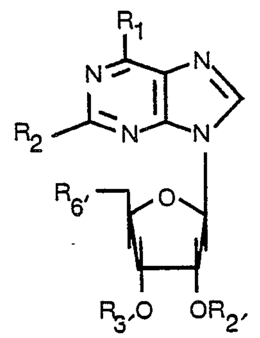

I

wherein $R_1$ is of formula

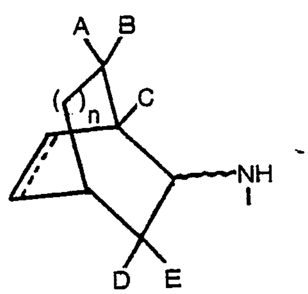

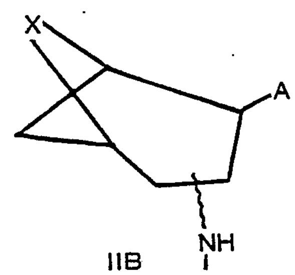

IIA IIB

in which ~NH— is either endo or exo; - - - - - is a double or single bond; n is zero, one, or two; A and B are either both hydrogen or both methyl; D and E are also either both hydrogen or both methyl; C is hydrogen or methyl; and with the proviso that when D and E are methyl then A and B are both hydrogen and C is methyl, but when D and E are hydrogen then A, B and C are all hydrogen or all methyl; X is $—C(CH_3)_2—$, $—CH_2—$, $—CH_2—CH_2—$, $—CH=CH—$. $R_2$ is hydrogen, halogen, SR where R is hydrogen or $C_1$ to $C_6$ alkyl, $NR^iR^{ii}$ where $R^i$ and $R^{ii}$ are independently hydrogen, $C_1$ to $C_6$ alkyl, phenyl or phenyl substituted by alkyl, $C_1$ to $C_6$ alkoxy, halogen, or trifluoromethyl; $R_2'$ and $R_3'$ are independently hydrogen, $C_1$ to $C_6$ alkanoyl, benzoyl, or benzoyl subsituted by $C_1$ to $C_6$ alkyl, $C_1$ to $C_6$ alkoxy, halogen or trifluoromethyl, or when taken together, $R_2'$ and $R_3'$ may be $C_1$ to $C_6$ alkylidene, such as isopropylidene; $R_6'$ is hydrogen, halogen or $R_5'O$ wherein $R_5'$ is hydrogen, $C_1$ to $C_6$ alkanoyl, benzoyl, or benzoyl substituted by $C_1$ to $C_6$ alkyl, $C_1$ to $C_6$ alkoxy, halogen or trifluoromethyl; and the ~NH— in the Formula IIB is attached to either one of the carbons adjacent; its individual diastereomers or mixtures thereof, or a pharmaceutically acceptable acid addition salt thereof.

The present invention also relates to a pharmaceutical composition comprising a therapeutically effective amount of a compound of the above Formula I with a pharmaceutically acceptable carrier, and to a method of treating mammals by administering to such mammals a dosage form of a compound of the Formula I as defined above.

In the compounds of the Formula 1, the terms "alkyl" and "$C_1$ to $C_6$ alkylidene" mean a straight or branched alkyl group or alkylidene radical having from 1 to 6 carbon atoms such as, for example, methyl, ethyl, propyl, isopropyl, butyl, *sec*-butyl, isobutyl, *tert*-butyl, amyl, isoamyl, neopentyl, hexyl, and the like.

Halogen includes particularly fluorine, chlorine or bromine.

$C_1$ to $C_6$ alkoxy is 0-alkyl from 1 to 6 carbon atoms as defined above for "$C_1$ to $C_6$ alkyl."

$C_1$ to $C_6$ alkanoyl is a straight or branched

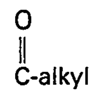

group of from 1 to 6 carbon atoms in the alkyl chain as defined above.

The term "and the ~NH— in the Formula IIB is attached to either one or the other of the adjacent carbons" means

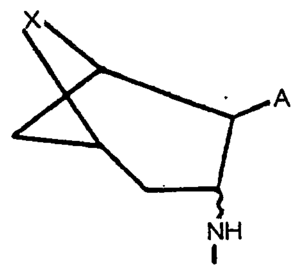

or

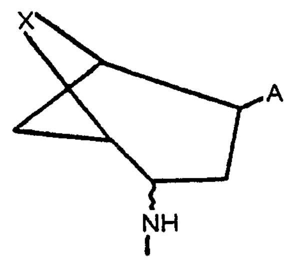

The compounds of Formula I are useful both in the free base form and in the form of acid addition salts. Both forms are within the scope of the invention. In practice, use of the salt form amounts to use of the base form. Appropriate pharmaceutically acceptable salts within the scope of the invention are those derived from mineral acids such as hydrochloric acid and sulfuric acid; and organic acids such as ethanesulfonic acid, benzenesulfonic acid, p-toluenesulfonic acid, and the like, giving the hydrochloride, sulfamate, ethanesulfonate, benzenesulfonate, p-toluenesulfonate, and the like, respectively.

The acid addition salts of said basic compounds are prepared either by dissolving the free base in aqueous alcohol solution or other suitable solvents containing the appropriate acid and isolating the salt by evaporating the solution, or by reacting the free base and acid in an organic solvent, in which case the salt separates directly or can be obtained by concentration of the solution.

The compounds of the invention may contain asymmetric carbon atoms. The invention includes the individual diastereomers in the $N^6$-substituent, the pure S, the pure R isomer, and mixtures thereof. The individual diastereomers may be prepared or isolated by methods known in the art.

Under certain circumstances it is necessary to protect either the N or O of intermediates in the above noted process with suitable protecting groups which are known. Introduction and removal of such suitable oxygen and nitrogen protecting groups are well known in the art of organic chemistry; see for example (1) "Protective Groups in Organic Chemistry," J. F. W. McOmie, ed., (New York, 1973), pp 43ff, 95ff; (2) J. F. W. McOmie, *Advances in Organic Chemistry,* Vol. 3, 191—281 (1963); (3) R. A. Borssonas, *Advances in Organic Chemistry,* Vol. 3, 159—190 (1963); and (4) J. F. W. McOmie, *Chem. & Ind.,* 603 (1979).

Examples of suitable oxygen protecting groups are benzyl, t-butyldimethysilyl, methyl, isopropyl, ethyl, tertiary butyl, ethoxyethyl, and the like. Protection of a N—H containing moiety is necessary for some of the processes described herein for the preparation of compounds of this invention. Suitable nitrogen protecting groups are benzyl, triphenylmethyl, trialkylsilyl, trichloroethylcarbamate, trichloroethoxcycarbonyl, vinyloxycarbamate, and the like.

Under certain circumstances it is necessary to protect two different oxygens with dissimilar protecting groups such that one can be selectively removed while leaving the other in place. The benzyl and t-butyldimethylsilyl groups are used in this way; either is removable in the presence of the other, benzyl being removed by catalytic hydrogenolysis, and t-butyldimethylsilyl being removed by reaction with, for example, tetra-n-butylammonium fluoride.

In the process described herein for the preparation of compounds of this invention the requirements for protective groups are generally well recognized by one skilled in the art of organic chemistry, and accordingly the use of appropriate protecting groups is necessarily implied by the processes of the charts herein, although not expressly illustrated.

The products of the reactions described herein are isolated by conventional means such as extraction, distillation, chromatography, and the like.

A preferred embodiment of the present invention is a compound of Formula I wherein $R_1$ is of Formula IIA; ~NH— is either endo or exo; A, B, C, D. and D are all hydrogen; $R_2$ is hydrogen, $R_6'$ is chloro; and $R_2'$ and $R_3'$ are as defined above.

A particular embodiment includes $N_6$-endo-bicyclo[2.2.1]heptyladenosine, $N_6$-exo-bicyclo[2.2.1]heptyl-adenosine, and $N_6$-endo-bicyclo[2.2.1]heptyl-5'-chloroadenosine. The $N_6$-endo-bicyclo[2.2.1]heptyl-5'-chloroadenosine is the most preferred embodiment.

The compounds of Formula I may be conveniently synthesized by reacting a 6-halopurine riboside of Formula III with bicyclo amine of Formula IVA or IVB as shown hereinafter where A, B, C, D, E, and X is as

defined above in an inert solvent such as alcohol, or an aprotic solvent such as dimethylformamide between about 25 to about 130°C for from 1—48 hours. The bicyclo amines of Formula IVA or Formula IVB above are commercially available, may be made by known methods or may be made by methods analogous to those known in the art. It is useful to add a base such as triethylamine, or calcium carbonate to neutralize the hydrogen halide formed as a byproduct of the reaction but this can also be accomplished by using an extra equivalent of the alkylamine. It is also convenient, although not necessary, to protect the ribofuranose hydroxyl groups as acetate or benzoate esters which can be removed with ammonium hydroxide or sodium methoxide following the synthesis of the $N^6$-substituted adenosine. The reaction is illustrated as follows:

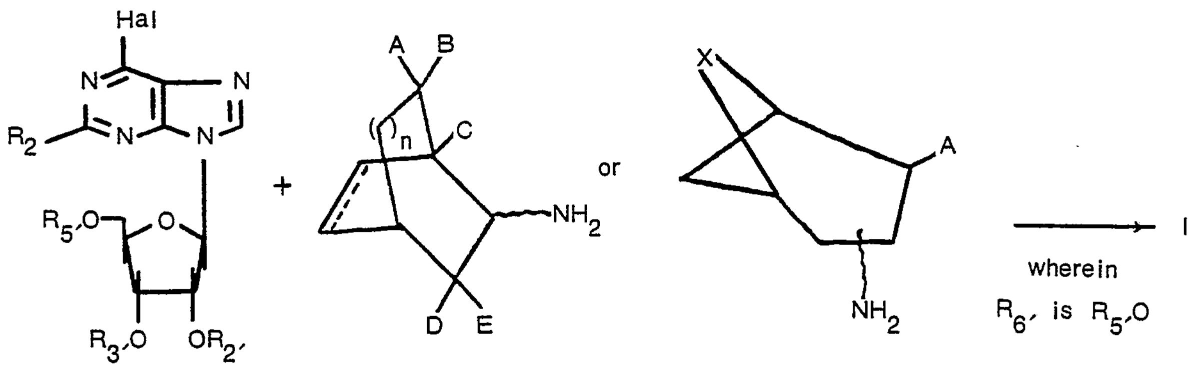


III IVA IVB

wherein Hal is halogen, preferably chlorine or bromine, and A, B, C, D, E, $R_2$, $R_2'$, $R_3'$, and $R_5'$ are as defined for Formula I.

In addition, compounds of Formula I wherein $R_6'$ is halogen may also be prepared from a compound Formula I where $R_6'$ is $R_5'O$ where $R_2'$, $R_3'$, and $R_5'$ are all hydrogen, in a stepwise manner, by first reacting with the compound to form a protecting group for the oxygen of $OR_2'$ and $OR_3'$, i.e., where $R_2'$ and $R_3'$ are other than hydrogen, e.g., dimethoxy propane, in acetone to give a compound of Formula I having protected groups followed by chlorinating the 5'-hydroxymethyl using thionyl chloride in tetrahydrofuran, and removing the protecting group under aqueous acid condition using acids, for example, hydrochloric, acetic, sulfuric, and the like as illustrated below.

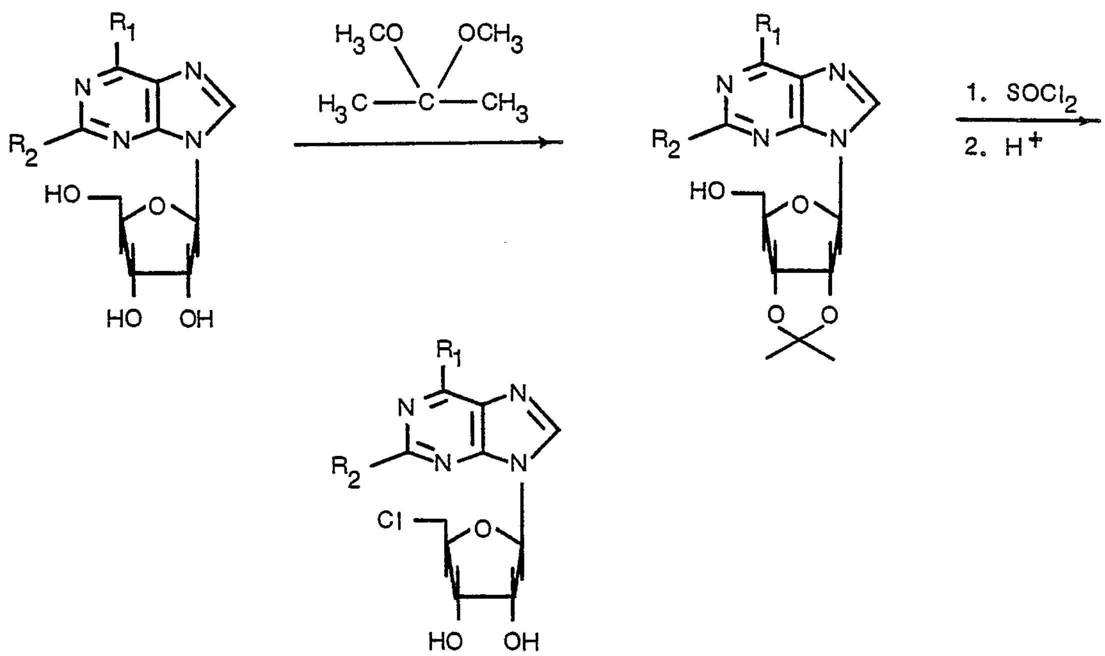


In addition, compounds of Formula I wherein $R_2$ is other than hydrogen or halogen, may also be prepared from 2,6-dichloropurine riboside triacetate of Formula V in a stepwise manner, by first reacting a compound of the Formula V with bicycloamine of Formula IVa or IVb to give a compound of Formula VI, followed by replacing the chlorine atom at $C_2$ with the group $R_2$ using nucleophilic displacement conditions, and removing a protecting group, i.e., the acetate, as illustrated below.

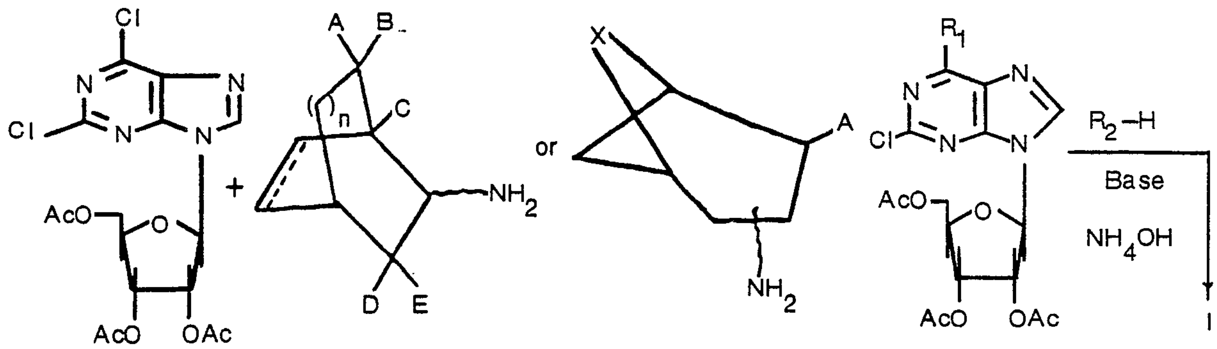


IVA IVB

The compounds of Formula I wherein $R_6'$ is hydrogen are prepared according to the following scheme:

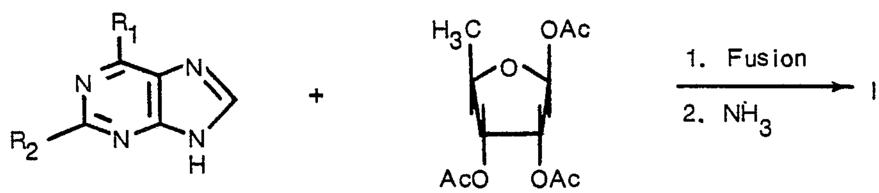


Generally the conditions of the above scheme are analogous to those known in the art.

The compounds of Formula I have been found to possess differing affinities for adenosine receptors (designated $A_1$ and $A_2$ receptors for convenience). These compounds are active in animal tests as having analgesic properties and as such, are useful in the treatment of pain and inflammation.

## Pharmacological Evaluation
## Adenosine Receptor Binding — $A_1$ Receptor Affinity (RBA1)

### Preparation of Membranes

Whole brain minus cerebellum and brainstem from male Long Evans rats (150—200 g) was homogenized in 30 volumes of ice-cold 0.05 M Tris-HCl buffer pH 7.7 using a Brinkman Polytron PT—10, (setting number 6 for 20 seconds) and centrifuged for ten minutes at 20,000 × g (Sorvall RC—2), 4°C. The supernatant was discarded, and the pellet was resuspended and centrifuged as before. The pellet was resuspended in 20 ml Tris-HCl buffer containing two International Units/ml of adenosine deaminase (Sigma type III from calf intestinal mucosa), incubated at 37°C for 30 minutes, then subsequently at 0°C for ten minutes. The homogenate was again centrifuged, and the final pellet was resuspended in ice-cold 0.05 M Tris-HCl buffer pH 7.7 to a concentration of 20 mg/ml original wet tissue weight and used immediately.

### Assay Conditions

Tissue homogenate (10 mg/ml) was incubated in 0.05 M Tris-HCl buffer pH 7.7 containing 1.0 nM [$^3$H]-$N^6$-cyclohexyladenosine ([$^3$H]-CHA) with or without test agents in triplicate for one hour at 25°C. Incubation volume was 2 ml. Unbound [$^3$H]-CHA was separated by rapid filtration under reduced pressure through Whatman glass fiber (GF/B) filters. The filters were rinsed three times with 5 ml of ice cold 0.05 M Tris-HCl buffer pH 7.7. The radiolabeled ligand retained on the filter was measured by liquid scintillation spectrophotometry after shaking the filters for one hour or longer on a mechanical shaker in 10 ml of Beckman Ready-Solv HP scintillation cocktail.

### Calculations

Nonspecific binding was defined as the binding which occurred in the presence of 1 mM theophylline. The concentration of test agent which inhibited 50% of the specific binding ($IC_{50}$) was determined by nonlinear computer curve fit. The Scatchard plot was calculated by linear regression of the line obtained by plotting the amount of radioligand bound (pmoles/gram of tissue) versus

$$\left[\frac{\text{bound radioligand}}{\text{free radioligand}}\right].$$

Since the amount of radioligand bound was a small fraction of the total amount added, free radioligand was defined as the concentration (nM) of radioligand added to the incubation mixture. The Hill coefficient was calculated by linear regression of the line obtained by plotting the log of the bound radioligand vs the log of the

$$\left[\frac{\text{bound radioligand}}{B_{max} - \text{bound radioligand}}\right].$$

The maximal number of binding sites ($B_{max}$) was calculated from the Scatchard plot.

Adenosine Receptor Binding — $A_2$ Receptor Affinity (RBA2)

Tissue Preparation

Brains from 200—500 g mixed sex Sprague-Dawley rats were purchased from Pel-Freez (Rogers, Arkansas). Fresh brains from male Long-Evans hooded rats (Blue Spruce Farms, Altamont, NY) gave essentially identical results. Brains were thawed out and then kept on ice while the striata were dissected out. Striata were disrupted in 10 vol of ice-cold 50 mM Tris·HCl (pH 7.7 at 25°C, pH 8.26 at 5°C) (Tris) for 30 seconds in a Polytron PT—10 (Brinkmann) at setting 5. The suspension was centrifuged at 50,000 xg for ten minutes, the supernatant discarded, the pellet resuspended in 10 vol ice-cold Tris as above, recentrifuged, resuspended at 1 g/5 ml, and stored in plastic vials at −70°C (stable for at least six months). When needed, tissue was thawed at room temperature, disrupted in a Polytron, and kept on ice until used.

Incubation Conditions

All incubations were for 60 minutes at 25°C in 12 × 75 mm glass tubes containing 1 ml Tris with 5 mg original tissue weight of rat weight of rat striatal membranes, 4 nM [$^3$H]-N-ethyl adenosine-5'-carboxamide ([$^3$H]NECA), 50 nM $N^6$-cyclopentyladenosine (to eliminate $A_1$ receptor binding), 10 mM $MgCl_2$, 0.1 units/ml of adenosine deaminase and 1% dimethylsulfoxide. $N^6$-Cyclopentyladenosine was dissolved at 10mM in 0.02 N Hcl and diluted in Tris. Stock solutions and dilutions of $N^6$-cyclopentyladenosine could be stored at −20°C for several months. Test compounds were dissolved at 10mM in dimethylsulfoxide on the same day as the experiment, and diluted in dimethylsulfoxide to 100x the final incubation concentration. Control incubations received an equal volume (10 µl) of dimethylsulfoxide; the resulting concentration of dimethylsulfoxide had no effect on binding. [$^3$H]NECA was diluted to 40 nM in Tris. The membrane suspension (5 mg/0.79 ml) contained sufficient $MgCl_2$ and adenosine deaminase to give 10 mM and 0.1 units/ml, respectively, final concentration in the incubation. For test compounds with $IC_{50}$ values less than 1 I µM, the order of additions was test compound (10 µl), $N^6$-cyclopentyladenosine (100 µl), [$^3$H)NECA (100 µl), and membranes (0.79 ml). For test compounds with $IC_{50}$ values greater than 1 µM and limited water solubility, the order of additions (same volumes) was test compound, membranes, $N^6$-cyclopentyladenosine, and [$^3$H]NECA. After all additions, the rack of tubes was vortexed, and the tubes were then incubated for 60 min at 25°C in a shaking water bath. The rack of tubes was vortexed an additional time halfway through the incubation.

Incubations were terminated by filtration through 2.4 cm GF/B filters under reduced pressure. Each tube was filtered as follows: the contents of the tube were poured onto the filter, 4 ml of ice-cold Tris were added to the tube and the contents poured onto the filter, and the filter was washed twice with 4 ml of ice-cold Tris. The filtration was complete in about twelve seconds. Filters were put in scintillation vials, 8 ml of Formula 947 scintillation fluid added, and the vials left overnight, shaken, and counted in a liquid scintillation counter at 40% efficiency.

Data Analysis

Nonspecific binding was defined as binding in the presence of 100 µM $N^6$-cyclopentyladenosine, and specific binding was defined as total binding minus nonspecific binding. The $IC_{50}$ was calculated by weighted nonlinear least squares curve-fitting to the mass-action equation.

$$Y = T - S \cdot \frac{D}{D + K}$$

where

Y is cpm bound

T is cpm total binding without drug

S is cpm specific binding without drug

D is the concentration of drug

and K is the $IC_{50}$ of the drug.

Weighting factors were calculated under the assumption that the standard deviation was proportional to the predicted value of Y. Nonspecific binding was treated as a very large (infinite) concentration of drug in the computer analysis.

The $IC_{50}$ values (nM) for adenosine $A_1$ and $A_2$ receptor affinity are reported in the table.

Analgesic Evaluation

The antiwrithing (AW) test provides preliminary assessment of compound with potential analgesic activity. The test is performed in male Swiss-Webster mice. Compounds are administered subcutaneously

in aqueous 0.2% methylcellulose or other appropriate vehicles in volumes of 10 ml/kg. Dosages represent active moiety.

Acetic acid (0.6%, 10 ml/kg) is injected intraperitoneally 20 minutes after administration of the adenosine agonist. Writhing movements are counted for five minutes starting seven minutes after the acetic acid injection. Writhing is defined as abdominal constriction and stretching of the body and hind legs with concave arching of the back. Data are expressed as $ED_{50}$ values, where the $ED_{50}$ is the dose necessary to suppress writhing by 50% relative to vehicle controls. $ED_{50}$ values are calculated by nonlinear regression analysis.

Antiinflammatory Assay

Assessment of immunoinflammatory or antiinflammatory activity is provided by the carrageenan pleurisy assay. Carrageenan pleurisy is induced as previously described by Carter, G. W. et al., in J. Pharm. Pharmacol 34:66—67, 1982. Carrageenan (310 μg/rat) is injected intrapleurally in a 0.25 ml volume of pyrogen-free saline. Four hours later, the rats are sacrificed and 2 ml of a phenol red solution (325 mg phenol red in 1 liter of 0.05 M phosphate buffered saline) are added to each pleural cavity. The contents of the cavities are mixed and transferred to glass test tubes. A 50 μl aliquot is removed from each tube and exudate cells are counted after red blood cells lysis (with Zapoglobin; Coulter Electronics, Hialeah FL) using a Coulter model ZBI counter. The remaining exudate-phenol red mixture is centrifuged at 750 xg for 15 minutes. One hundred μl of the supernatant fluid is diluted with 3.9 ml of phosphate buffer (0.072 M of tribasic sodium phosphate, $Na_3PO_4 \cdot 12H_2O$, in water) and the absorbance is measured at 560 nm.

Exudate volume is calculated as follows:

$$V_1 = \frac{V\,2\,(A_2 - A_3)}{(A_3 - A_1)}$$

where

$V_1$ = unknown volume of exudate,

$V_2$ = volume of dye added to cavity (2 ml),

$A_1$ = absorbance of the exudate (assumed to be zero),

$A_2$ = absorbance of the phenol red solution,

$A_3$ = absorbance of exudate and phenol red solution.

Inhibition of exudate or formation is calculated by the following equations:

$$\%\ \text{inhibition (exudate)} = \frac{\text{Vehicle Exudate Volume} - \text{Inhibitor Exudate}}{\text{Vehicle Exudate Volume}} \times 100$$

$$\%\ \text{inhibition (cell count)} = \frac{\text{Vehicle Cell Count} - \text{Inhibitor Cell Count}}{\text{Vehicle Cell Count}} \times 100$$

$ID_{50}$ values are calculated by Probit analysis.

The compound of Example 1 was administered one hour prior to carrageenan injection.

The biological data are summarised in the Table. Accordingly, the present invention also includes a pharmaceutical composition for treating pain, and inflammation comprising a corresponding analgesic or antiinflammatory effective amount of a compound of the Formula I as defined above together with a pharmaceutically acceptable carrier.

Receptor Binding

| Example | RBA-1 (nM) | RBA-2 (nM) |
|---|---|---|
| 1 | 0.85 | 1300 |
| 2 | 1.4 | 1400 |
| 5 | 0.69 | 3093 |

AW Test (Analgesic Test)

| Example | AW $ED_{50}$ mg/kg |
|---|---|
| 1 | 0.03 |

Carrageenan Pleurisy Assay
(Immunoinflammatory Test)

| Example | $ID_{50}$ (mg/kg) Exudate | WBC |
|---|---|---|
| 1 | 0.15 | 0.12 |

or specifically for various doses as follows:

Carrageenan Pleurisy Assy

| Example | Dose mg/kg | % Inhibition Exudate | WBC |
|---|---|---|---|
| 1 | 0.1 | 49.3 | 74.1 |
| | 0.2 | 60.0 | 70.0 |
| | 0.4 | 81.3 | 90.0 |
| | 0.8 | 42.7 | 68.4 |
| 2 | 0.3 | 24.3 | 25.7 |
| | 1.0 | 22.9 | 52.9 |
| 5 | 0.1 | 11.7 | 18.4 |
| | 0.3 | 16.8 | 40.0 |

The present invention further includes a method for treating pain or inflammation in mammals suffering therefrom comprising administering to such mammals either orally or parenterally a corresponding pharmaceutical composition containing a compound of the Formula I as defined above in appropriate unit dosage form.

For preparing pharmaceutical compositions from the compounds described by this invention, inert, pharmaceutically acceptable carriers can be either solid or liquid. Solid form preparations include powders, tablets, dispersible granules, capsules, cachets, and suppositories. A solid carrier can be one or more

substances which may also act as diluents, flavoring agents, solubilizers, lubricants, suspending agents, binders or tablet disintegrating agents; it can also be encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 to 10 to about 70 percent of the active ingredient. Suitable solid carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component (with or without other carrier) is surrounded by carrier, which is thus in association with it. Similarly, cachets are included. Tablets, powders, cachets, and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogenous mixture is then poured into convenient sized molds, allowed to cool and thereby to solidify.

Liquid form preparations include solutions, suspensions, and emulsions. As an example may be mentioned water or water propylene glycol solutions for parenteral injection. Liquid preparations can also be formulated in solution in aqeuous polyethylene glycol solution. Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, i.e., natural or synthetic gums, resins, methylcellulose, sodium carboxymethylcellulose, and other well-known suspending agents.

Also included are solid form preparations which are intended to be converted, shortly before use, to liquid form preparations for either oral or parenteral administration. Such liquid forms include solutions, suspensions, and emulsions. These particular solid form preparations are most conveniently provided in unit dose form and as such are used to provide a single liquid dosage unit. Alternately, sufficient solid may be provided so that after conversion to liquid form, multiple individual liquid doses may be obtained by measuring predetermined volumes of the liquid form preparation as with a syringe, teaspoon, or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavorants, colorants, stabilizers, buffers, artificial and natural sweeteners, dispersants, thickeners, solubilizing agents, and the like. The liquid utilized for preparing the liquid form preparation may be water, isotonic water, ethanol, glycerine, propylene glycol, and the like as well as mixtures thereof. Naturally, the liquid utilized will be chosen with regard to the route of administration, for example, liquid preparations containing large amounts of ethanol are not suitable for parenteral use.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules, and powders in vials or ampoules. The unit dosage form can also be a capsule, cachet, or tablet itself, or it can be the appropriate number of any of these in packaged form.

The quantity of active compound in a unit dose of preparation may be varied or adjusted from 1 mg to 500 mg preferably to 5 to 100 mg according to the particular application and the potency of the active ingredient. The compositions can, if desired, also contain other compatible therapeutic agents.

In therapeutic use as described above, the mammalian dosage range for a 70 kg subject is from 0.1 to 150 mg/kg of body weight per day or preferably 1 to 50 mg/kg of body weight per day. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter the dosage is increased by small increments until the optimum effect under the circumstances is reached. For convenience, the total daily dosage may be divided and administered in portions during the day if desired.

The following Examples further illustrate the invention.

Example 1

N6-endo-Bicyclo[2.2.1]heptyl adenosine

A mixture of 4.0 g of 6-chloropurine riboside, 2.57 g endo-2-aminonoborane hydrochloride and 3.53 g triethylamine is refluxed in 100 ml ethanol under nitrogen for 20 hours. The solvent is evaporated to dryness. Residual solid material is dissolved in minimum of 2-propanol and diluted with excess cold water. Clear aqueous solution is decanted off. The residual solid is dissolved in ethanol and volatiles are evaporated. This is repeated once more yielding 3.8 g (75%) of N-6-endobicyclo[2.2.1]heptyladenosine having a melting point of 128—130°C.

| | | | |
|---|---|---|---|
| Analysis calculated for $C_{17}H_{23}N_5O_4$: | C, 56.49; | H, 6.41; | N, 19.37; |
| Found: | C, 56.44; | H, 6.81; | N, 18.78 |

Example 2

N6-exo-Bicyclo[2.2.1]heptyladenosine

A mixture of 4.0 g of 6-chloropurine riboside, 1.8 g exo-2-aminonoborane and 2.1 g of triethylamine is refluxed in 100 ml ethanol under nitrogen for 20 hours. The solvent is evaporated to dryness. The residual solid is treated with 50 ml of cold $H_2O$. Clear aqueous solution is decanted off and the solid material is dissolved in ethanol. Volatiles are evaporated under vacuo. This is repeated twice yielding solid material which is crystallized from ethanol-ethyl acetate-hexane affording 4.1 g (81%) of N-6-exo-bicyclo[2.2.1]heptyladenosine having a melting point of 110—112°C.

| | | | | |
|---|---|---|---|---|
| Analysis calculated for $C_{17}H_{23}N_5O_4$: | C, 56.49; | H, 6.41; | N, 19.37; | |
| Found: | C, 55.88; | H, 5.89; | N, 18.59 | |

Example 3

N6-2-(endo)-norbornyl-2′,3′-O-isopropylidene adenosine

N6-2-(endo)norbornyladenosine (12.7 g, 35 mmol), 2,2-dimethoxy propane (35 ml) and bis-p-nitrophenyl-phosphate hydrate (12.8 g, 38 mmol) were stirred in acetone (150 ml) at room temperature 18 hours. The reaction was quenched with 0.1 N $NaHCO_3$ (100 ml) and stirred for one hour. The acetone was evaporated in vacuo and the aqueous solution extracted with methylene chloride (200 ml). The organics were dried over magnesium sulfate and evaporated in vacuo to give off white foam. The foam was dissolved in methanol (100 ml) and poured through a plug of Dowex 1 × 8 ($NaHCO_3$ form) resin. The resin was washed with methanol (300 ml) and the combined filtrates evaporated in vacuo go give 10.6 g (75%) of a white solid, mp 90—98°C.

| | | | |
|---|---|---|---|
| Analysis calculated for $C_{10}H_{27}N_5O_4$: | C, 59.84; | H, 6.78; | N, 17.45; |
| Found: | C, 59.48; | H, 6.71; | N, 17.24 |

Example 4

N6-2-(endo)-norbornyl-5′-chloro-5′-deoxy-2′,3′-O-isopropylidene adenosine

The isopropylidene analog, as prepared in Example 3 above, (6.3 g, 15.7 mmol) was stirred in THF (100 ml) and treated with thionylchloride (2.8 g, 23.5 mmol). The reaction stirred at room temperature overnight. The solvent was evaporated in vacuo and the residue dissolved in methylene chloride (100 ml), and washed with water (2 × 100 ml). The organic dried over magnesium sulfate and evaporated in vacuo. The residue dissolved in ethyl acetate (25 ml) and purified by prep 500A chromatography (silica gel, 1 column, 100 ml/min). The slow running fraction was isolated by evaporation of solvent to give 4.4 g (67%) of a white foam, mp 61—70°C.

| | | | | |
|---|---|---|---|---|
| Analysis calculated for $C_{20}H_{26}ClN_5O_3$: | C, 57.20; | H, 6.24; | N, 16.68; | Cl, 8.44 |
| Found: | C, 56.97; | H, 6.13; | N, 16.59; | Cl, 8.62 |

Example 5

N6-2-endo-norbornyl-5′-chloro-5′-deoxy-adenosine

The 5′-chloro-isopropylidene analog, as prepared in Example 4 above, (4.3 g, 10.2 mmol) was stirred in 50% formic acid (100 ml) at 50°C for four hours. The acid was evaporated in vacuo and the residue coevaporated with methanol (2 × 50 ml) to give an off-white foam. The foam was dissolved in acetone (25 ml) and purified by prep 500A chromatography (silica gel, 1 column, 100 ml/min). The major refraction index active fraction was isolated by evaporation of solvent to give 2.4 g (62%) of a white solid, mp 96—99°C.

| | | | |
|---|---|---|---|
| Analysis calculated for $C_{17}H_{22}ClN_5O_3$: | C, 53.75; | H, 5.84; | N, 18.44; |
| Found: | C, 53.89; | H, 6.05; | N, 18.23 |

Example 6

$N^6$-(endo)norbornyl-5′-deoxyadenosine

Six chloropurine (2.3 g, 15 mmol), triethyl amine (3.5 g, 35 mmol) and 2-endo-aminonorbornane hydrochloride (2.5 g, 17 mmol) were stirred at reflux in 100 ml of ethanol for 72 hours. The solution was cooled to room temperature and the ethanol was evaporated in vacuo. The residue was dissolved in chloroform and washed with water (100 ml). The organics were dried over magnesium sulfate and the solvent was evaporated in vacuo. The residue was dried at 65°C in vacuo overnight to give 2.1 g (62%) of a light green solid, mp 217—219°C.

The adenine (4.4 g, 19.1 mmol) and 5′ deoxy ribose (6.1 g, 23 mmol) were melted and stirred at 200°C. To the melt was added one micro drop of concentrated sulfuric acid and the acetic acid formed was removed in a gentle stream of nitrogen. The solution was stirred for four hours before cooling to room temperature. The glassy residue was broken up in 100 ml of ethyl acetate in an ultrasonic bath. The solution was then purified by chromatography to give after evaporation of the solvent 1.6 g of an off-white foam, identified in Example 7 hereinafter. The foam (1.4 g, 3.3 mmol) was then dissolved in 100 ml of methanolic ammonia (saturated at 0°C) and the solution was stirred at room temperature for six hours. The solution was evaporated in vacuo and the residue purified by chromatography to give, after evaporation of solvent 0.9 g (18%) of a hygroscopic white solid, mp 93—101°C.

Example 7

$N^6$-Endo-norbornyl-5'-deoxyadenosine-2',3'-di-O-acetyl

Compound 7 from Example 6 was analyzed to give the product $N^6$-(2-endo-norbornyl)-5'-deoxy-2',3'-diacetyl adenosine 1.6 g (19.5%), mp 68—77°C.

| | | | |
|---|---|---|---|
| Analysis calculated for $C_{21}H_{27}N_5O_5$: | C, 58.34; | H, 6.60; | N, 14.79; |
| Found: | C, 58.32; | H, 6.48; | N, 14.86 |

**Claims for the Contracting States: BE CH DE FR GB IT LI LU NL SE**

1. A compound of the formula (I)

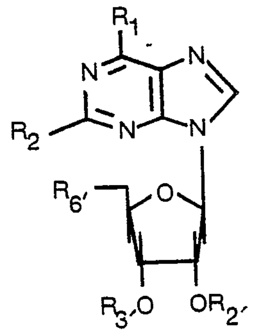

I

its individual diastereomers or mixtures thereof, or a pharmaceutically acceptable acid addition salt thereof;
wherein $R_1$ is of formula

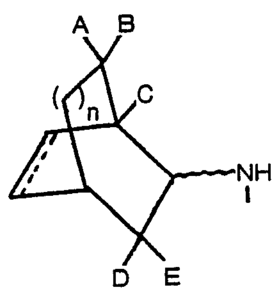

IIA

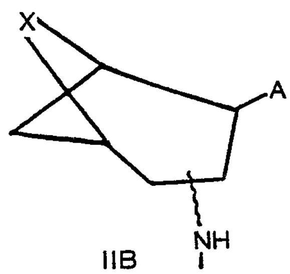

IIB

in which

NH— is either endo or exo;

----- is a double or single bond;

n is zero, one, or two;

A and B are either both hydrogen or both methyl; D and E are also either both hydrogen or both methyl; C is hydrogen or methyl; with the proviso that when D and E are methyl then A and B are both hydrogen and C is methyl, but when D and E are hydrogen then A, B and C are all hydrogen or all methyl;

X is $-C(CH_3)_2-$, $-CH_2-$, $-CH_2CH_2-$, $-CH=CH-$;

$R_2$ is hydrogen, halogen, SR where R is hydrogen or $C_1$ to $C_6$ alkyl, $NR^iR^{ii}$ where $R^i$ and $R^{ii}$ are independently hydrogen, $C_1$ to $C_6$ alkyl, phenyl or phenyl substituted by alkyl, $C_1$ to $C_6$ alkoxy, halogen, or trifluoromethyl;

$R_2'$ and $R_3'$ are independently hydrogen, $C_1$ to $C_6$ alkanoyl, benzoyl, or benzoyl substituted by $C_1$ to $C_6$ alkyl, $C_1$ to $C_6$ alkoxy, halogen or trifluoromethyl, or, when taken together, $R_2'$ and $R_3'$ may be $C_1$ to $C_6$ alkylidene; and

$R_6'$ is halogen, hydrogen or $R_5'O$ wherein $R_5'$ is hydrogen, $C_1$ to $C_6$ alkanoyl, benzoyl, or benzoyl 5 substituted by $C_1$ to $C_6$ alkyl, $C_1$ to $C_6$ alkoxy, halogen or trifluoromethyl; and the NH— is attached to either one or the other of the adjacent carbons in the radical IIB.

2. A compound as claimed in Claim 1, wherein $R_2$ is hydrogen.

3. A compound as claimed in Claim 1, wherein $R_1$ is of the Formula IIA.

4. A compound as claimed in Claim 1, wherein $R_1$ is of the Formula IIB.

5. A compound as claimed in Claim 3 and having one of the following names:

$N^6$-endo-bicyclo[2.2.1]heptyladenosine;

$N^6$-exo-bicyclo[2.2.1]heptyladenosine;

endo-bicyclo[2.2.1]heptyl-5'-chloro-5'-deoxyadenosine;

$N^6$-(endo)-norbornyl-5'-deoxyadenosine;
$N^6$-(2-endo-norbornyl)-5'-deoxy-2',3'-diacetyladenosine;
$N^6$-norbornyl-2',3'-O-isopropylideneadenosine; and
$N^6$-2-(endo)-norbornyl-5'-chloro-5'-deoxy-2',3'-isopropylidene adenosine.

6. A process for the preparation of a compound as claimed in Claim 1, which comprises reacting a 6-halopurine riboside of the formula

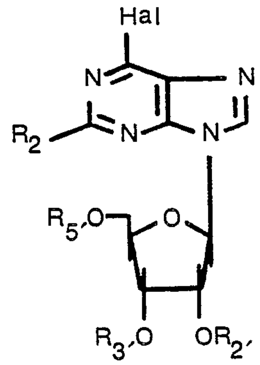

III

wherein Hal is halogen and $R_2$, $R_2'$, $R_3'$, $R_5'$ are as defined in Claim 1, with a compound of formula:

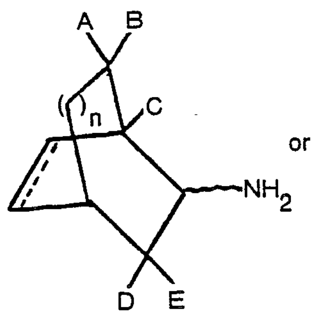

or

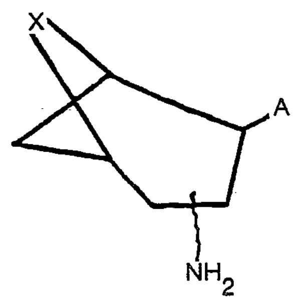

IVA IVB

in an inert solvent at about 25 to abut 130°C for from 1—48 hours, and, if desired, converting $R_5'O$ to halogen or methyl; and, further, if desired, converting the resulting free base to a pharmaceutically acceptable acid addition salt.

7. A process for the preparation of a compound as claimed in Claim 1 in which $R_2$ is SR or $NR^iR^{ii}$ and $R_2'$, $R_3'$, and $R_5'$ are hydrogen, which process comprises reacting a compound of the formula

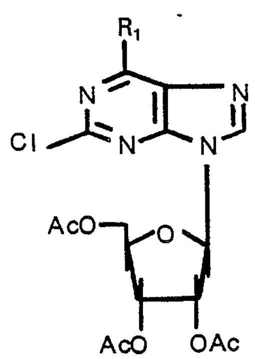

in which $R_1$ is as defined in Claim 1 and wherein Ac is acetyl, with a nucleophile corresponding to $R_2$; removing the acetyl groups with ammonium hydroxide, and, if desired, converting the resulting free base to a pharmaceuticaly acceptable acid addition salt.

8. A process for the preparation of a compound as claimed in Claim 1 in which $R_6'$ is chloro, which process comprises reacting a compound of the formula

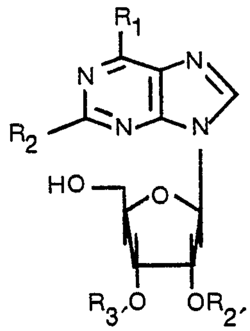

wherein $R_1$ and $R_2$ are as defined above, and $R_3'$ and $R_2'$ are as defined above with the additional proviso that neither are hydrogen; with thionyl chloride, and, if desired, making $R_3'$ and $R_2'$ each hydrogen by further treatment with an aqueous acid, and, if desired, converting the resulting free base to a pharmaceutically acceptable acid addition salt.

9. A process for the preparation of a compound as claimed in Claim 1 in which $R_6'$ is hydrogen, which process comprises reacting a compound of the formula

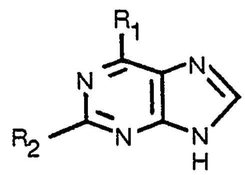

wherein $R_1$ and $R_2$ are as defined above with a compound of the formula

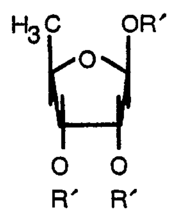

wherein R′ is a protecting group.

10. A pharmaceutical composition comprising a therapeutically effective amount of a compound as claimed in any one of Claims 1 to 5 together with a pharmaceutically acceptable carrier or diluent.

11. For use in a method of treating pain in a mammal suffering therefrom, a compound as claimed in any one of Claims 1 to 5, preferably in unit dosage form.

12. For use in a method of treating inflammation in a mammal suffering therefrom, a compound as claimed in any one of Claims 1 to 5, preferably in unit dosage form.

**Claims for the Contracting State: AT**

1. A process for preparing a compound of the formula (I)

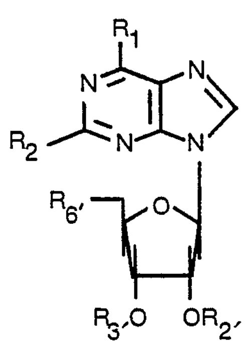

I

its individual diastereomers or mixtures thereof, or a pharmaceutically acceptable acid addition salt thereof; wherein $R_1$ is of formula

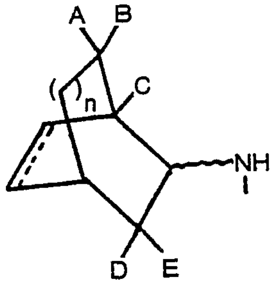

IIA

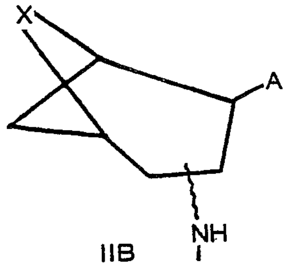

IIB

in which

NH— is either endo or exo;

- - - - - is a double or single bond;

n is zero, one, or two;

A and B are either both hydrogen or both methyl; D and E are also either both hydrogen or both methyl; C is hydrogen or methyl; with the proviso that when D and E are methyl then A and B are both hydrogen and C is methyl, but when D and E are hydrogen then A, B and C are all hydrogen or all methyl;

X is $—C(CH_3)_2—$, $—CH_2—$, $—CH_2CH_2—$, or —CH=CH—;

$R_2$ is hydrogen, halogen, SR where R is hydrogen or $C_1$ to $C_6$ alkyl, NR′R″ where R′ and R″ are independently hydrogen, $C_1$ to $C_6$ alkyl phenyl or phenyl substituted by alkyl, $C_1$ to $C_6$ alkoxy, halogen, or trifluoromethyl;

$R_2'$ and $R_3'$ are independently hydrogen, $C_1$ to $C_6$ alkanoyl, benzoyl, or benzoyl substituted by $C_1$ to $C_6$ alkyl, $C_1$ to $C_6$ alkoxy, halogen or trifluoromethyl, or, when taken together, $R_2'$ and $R_3'$ may be $C_1$ to $C_6$ alkylidene; and

$R_6'$ is halogen, hydrogen or $R_5'O$ wherein $R_5'$ is hydrogen, $C_1$ to $C_6$ alkanoyl, benzoyl, or benzoyl substituted by $C_1$ to $C_6$ alkyl, $C_1$ to $C_6$ alkoxy, halogen or trifluoromethyl; and the NH— is attached to either one or the other of the adjacent carbons in the radical IIB; which process comprises reacting a 6-halopurine riboside of the formula

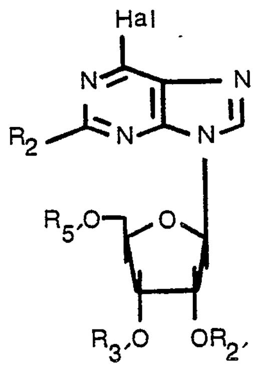

III

wherein Hal is halogen and $R_2$, $R_2'$, $R_3'$, $R_5'$ are as defined above, with a compound of formula:

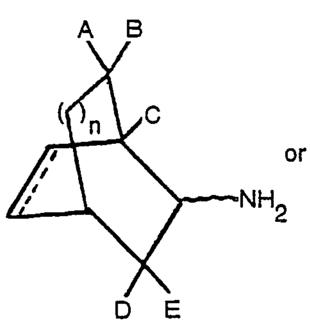

or

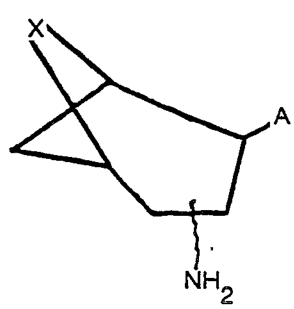

IVA IVB

in an inert solvent at about 25 to about 130°C for from 1—48 hours, and, if desired, converting $R_5'O$ to halogen or methyl; and, further, if desired, converting the resulting free base to a pharmaceutically acceptable acid addition salt.

2. A process for the preparation of a compound of formula I as defined in Claim 1 in which $R_2$ is SR or $NR^IR^{II}$ and $R_2'$, $R_3'$, and $R_5'$ are hydrogen, which process comprises reacting a compound of the formula

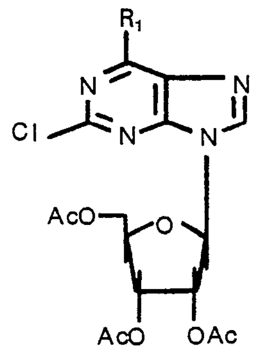

in which $R_1$ is as defined in Claim 1 and wherein Ac is acetyl, with a nucleophile corresponding to $R_2$; removing the acetyl groups with ammonium hydroxide, and, if desired, converting the resulting free base to a pharmaceutically acceptable acid addition salt.

3. A process for the preparation of a compound of formula I as defined in Claim 1 in which $R_6'$ is chloro, which process comprises reacting a compound of the formula

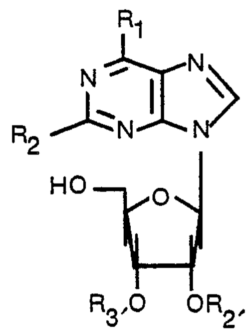

wherein $R_1$ and $R_2$ are as defined above, and $R_3'$ and $R_2'$ are as defined above with the additional proviso that neither are hydrogen; with thionyl chloride, and, if desired, making $R_3'$ and $R_2'$ each hydrogen by further treatment with an aqueous acid, and, if desired, converting the resulting free base to a pharmaceutically acceptable acid addition salt.

4. A process for the preparation of a compound of formula I of Claim 1 in which $R_6'$ is hydrogen, which process comprises reacting a compound of the formula

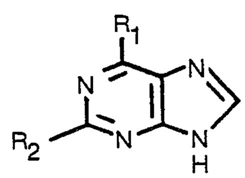

wherein $R_1$ and $R_2$ are as defined above with a compound of the formula

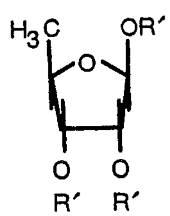

wherein R′ is a protecting group.

5. A process according to any preceding claim, wherein $R_2$ is hydrogen.

6. A process according to any one of Claims 1 to 4 wherein $R_1$ is of the Formula IIA.

7. A process according to any one of Claims 1 to 4 wherein $R_1$ is of the Formula IIB.

8. A process according to Claim 6 in which one of the following compounds is prepared:

$N^6$-endo-bicyclo[2.2.1]heptyladenosine;

$N^6$-exo-bicyclo[2.2.1]heptyladenosine;
endo-bicyclo[2.2.1]heptyl-5'-chloro-5'-deoxyadenosine;
$N^6$-(endo)-norbornyl-5'-deoxyadenosine;
$N^6$-(2-endo-norbornyl)-5'-deoxy-2',3'-diacetyladenosine;
$N^6$-norbornyl-2',3'-O-isopropylideneadenosine; and
$N^6$-2-(endo)-norbornyl-5'-chloro-5'-deoxy-2',3'-isopropylidene adenosine.

9. A process for preparing a pharmaceutical composition which comprises combining a therapeutically effective amount of a compound prepared by a process as claimed in any one of Claims 1 to 5 together with a pharmaceutically acceptable carrier or diluent.

10. For use in a method of treating pain in a mammal suffering therefrom, a compound prepared by a process as claimed in any one of Claims 1 to 8, preferably in unit dosage form.

11. For use in a method of treating inflammation in a mammal suffering therefrom, a compound prepared by a process as claimed in any one of Claims 1 to 8, preferably in unit dosage form.

**Patentansprüche für die Vertragsstaaten: BE CH DE FR GB IT LI LU NL SE**

1. Verbindung der Formel (I)

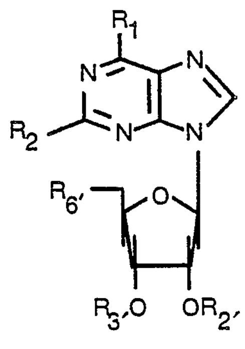

I

ihre einzelnen Diastereomeren oder Mischungen davon oder ein pharmazeutisch akzeptables Säureadditionssalz davon;
worin
$R_1$ ist: die Formel

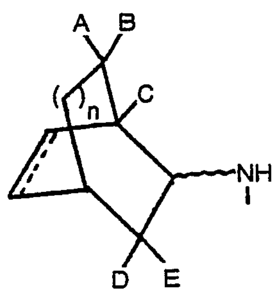

IIA

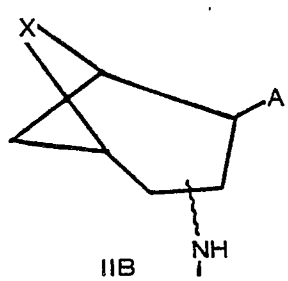

IIB

worin
NH— entweder für endo oder exo steht;
- - - - - eine Doppel- oder Einfachbindung darstellt;
n für Null, Eins oder Zwei steht;
A und B entweder beide Wasserstoff oder beide Methyl sind;
D und E ebenso entweder beide Wasserstoff oder beide Methyl repräsentieren;
C für Wasserstoff oder Methyl steht;
mit der Maßgabe, daß wenn D und E Methyl bedeuten, A und B beide Wasserstoff sind, und C für Methyl steht, aber wenn D und E Wasserstoff sind, A, B und C alle Wasserstoff oder alle Methyl sind;
X bedeutet: $—C(CH_3)_2—$, $—CH_2—$, $—CH_2CH_2—$ oder $—CH{=}CH—$;
$R_2$ ist: Wasserstoff, Halogen, SR, worin R für Wasserstoff oder Alkyl mit 1 bis 6 Kohlenstoffatomen steht, $NR^IR^{II}$, worin $R^I$ und $R^{II}$ unabhängig voneinander sind: Wasserstoff, Alkyl mit 1 bis 6 Kohlenstoffatomen, Phenyl oder Phenyl substituiert durch Alkyl mit 1 bis 6 Kohlenstoffatomen, Alkoxy mit 1 bis 6 Kohlenstoffatomen, Halogen oder Trifluoromethyl;
$R_2'$ und $R_3'$ unabhängig voneinander sind: Wasserstoff, Alkanoyl mit 1 bis 6 Kohlenstoffatomen, Benzoyl oder Benzoyl substituiert durch Alkyl mit 1 bis 6 Kohlenstoffatomen, Alkoxy mit 1 bis 6 Kohlenstoffatomen, Halogen oder Trifluormethyl, oder wenn sie zusammengenommen werden, $R_2'$ und $R_3'$ Alkyliden mit 1 bis 6 Kohlenstoffatomen sein können; und

$R_6'$ ist: Halogen, Waserstoff oder $R_5'O$, worin $R_5'$ darstellt: Wasserstoff, Alkanoyl mit 1 bis 6 Kohlenstoffatomen, Benzoyl oder Benzoyl substituiert durch Alkyl mit 1 bis 6 Kohlenstoffatomen, Alkoxy mit 1 bis 6 Kohlenstoffatomen, Halogen oder Trifluormethyl; und das NH— an das eine bzw. das andere der angrenzenden Kohlenstoffatome im Radikal IIB gebunden ist.

2. Verbindung wie im Anspruch 1 beansprucht, worin $R_2$ Wasserstoff ist.

3. Verbindung wie im Anspruch 1 beansprucht, worin $R_1$ die Formel IIA hat.

4. Verbindung wie im Anspruch 1 beansprucht, worin $R_1$ die Formel II B hat.

5. Verbindung wie im Anspruch 3 beansprucht, welche einen der folgenden Namen hat:

$N^6$-endo-Bicyclo[2.2.1]heptyladenosin;
$N^6$-exo-Bicyclo[2.2.1]heptyladenosin;
endo-Bicyclo[2.2.1]heptyl-5'-chloro-5'-desoxyadenosin;
$N^6$-(endo)-Norbornyl-5'-desoxyadenosin;
$N^6$-(2-endo-Norbornyl)-5'-desoxy-2',3'-diacetyladenosin;
$N^6$-Norbornyl-2',3'-O-isopropylidenadenosin; und
$N^6$-2-(endo)-Norbornyl-5'-chloro-5'-desoxy-2',3'-isopropylidenadenosin.

6. Verfahren zur Herstellung einer Verbindung wie im Anspruch 1 beansprucht, welches umfaßt: die Umsetzung eines 6-Halogenpurinribosids der Formel

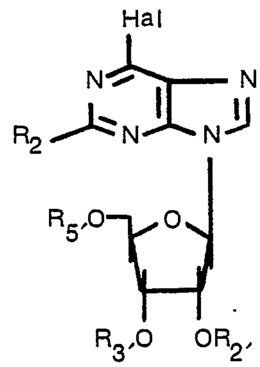

III

worin Hal für Halogen steht, und $R_2$, $R_2'$, $R_3'$, $R_5'$ wie im Anspruch 1 definiert sind, mit einer Verbindung der Formel:

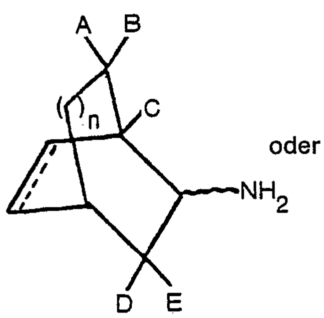

IVA

oder

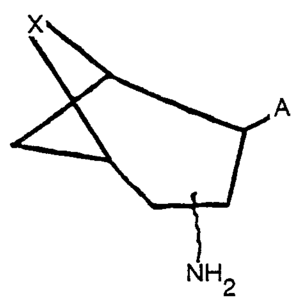

IVB

in einem inerten Lösungsmittel bei etwa 25 bis etwa 130°C von 1 bis 48 Stunden lang, und gewünschtenfalls Überführen von $R_5'O$ in Halogen oder methyl; und weiters, gewünschtenfalls Überführen der resultierenden freien Base in ein pharmazeutisch akzeptables Säureadditionssalz.

7. Verfahren zur Herstellung einer Verbindung wie im Anspruch 1 beansprucht, worin $R_2$ für SR oder $NR^IR^{II}$ steht, und $R_2'$, $R_3'$ und $R_5'$ Wasserstoff darstellen, welches Verfahren umfaßt: die Reaktion einer Verbindung der Formel

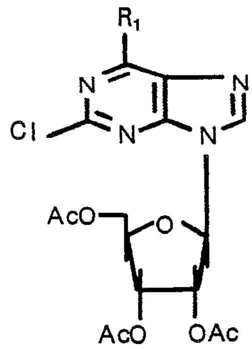

worin $R_1$ wie im Anspruch 1 definiert ist, und worin Ac für Acetyl steht, mit einem Nukleophil, welches $R_2$ entspricht; das Entfernen der Acetylgruppen mit Ammoniumhydroxid und gewünschtenfalls das Überführen der resultierenden freien Base in ein pharmazeutisch akzeptables Säureadditonssalz.

8. Verfahren zur Herstellung einer Verbindung wie im Anspruch 1 beansprucht, worin $R_6'$ für Chlor steht, welches Verfahren umfaßt:
die Umsetzung einer Verbindung der Formel

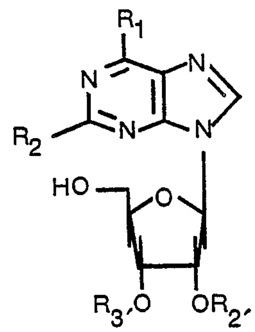

worin $R_1$ und $R_2$ wie oben definiert sind, und $R_3'$ und $R_2'$ wie oben definiert sind, mit der zusätzlichen Maßgabe, daß keines davon Wasserstoff bedeutet; mit Thionylchlorid und gewünschtenfalls die Bildung von sowohl $R_3'$ als auch $R_2'$ als Wasserstoff durch weitere Behandlung mit einer wässerigen Säure und gewünschtenfalls das Überführen der resultierenden freien Base in ein pharmazeutisch akzeptables Säureadditionssalz.

9. Verfahren zur Herstellung einer Verbindung wie im Anspruch 1 beansprucht, worin $R_6'$ Wasserstoff ist, welches Verfahren umfaßt:
die Umsetzung einer Verbindung der Formel

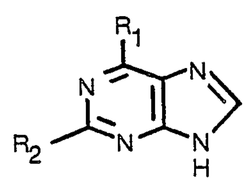

worin $R_1$ und $R_2$ wie oben definiert sind, mit einer Verbindung der Formel

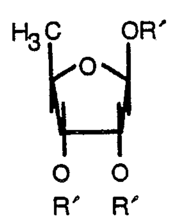

worin R' eine Schutzgruppe ist.

10. Pharmazeutische Zusammensetzung, welche eine therapeutisch wirksame Menge einer wie in irgendeinem der Ansprüche 1 bis 5 beanspruchten Verbindung zusammen mit einem Pharmazeutisch akzeptablen Träger oder Verdünnungsmittel umfaßt.

11. Verbindung, wie in irgendeinem der Ansprüche 1 bis 5 beansprucht, für die Verwendung in einer Methode zur Behandlung von Schmerz bei eine daran leidenden Säuger, vorzusweise in Dosiseinheitsform.

12. Verbindung, wie in irgendeinem der Ansprüche 1 bis 5 beansprucht, für die Verwendung in einer Methode zur Behandlung von Entzündung bei einem daran leidenden Säuger, vorzugsweise in Dosiseinheitsform.

**Patentansprüche für den Vertragsstaat: AT**

1. Verfahren zur Herstellung einer Verbindung der Formel (I)

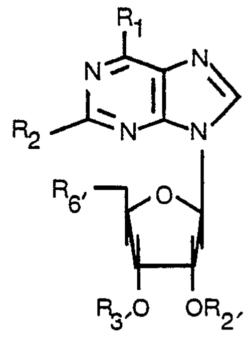

I

ihre einzelnen Diastereomeren oder von Mischungen davon oder eines pharmazeutisch akzeptablen Säureadditionssalzes davon,
worin
$R_1$ ist: die Formel

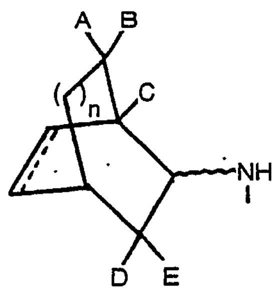

IIA

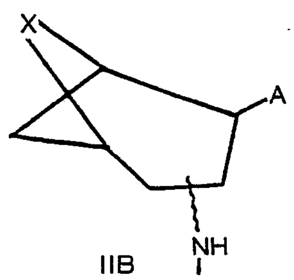

IIB

worin
NH— entweder für endo oder exo steht;
----- ein Doppel- oder Einfachbindung ist;
n für Null, Eins oder Zwei steht;
A und B entweder beide Wasserstoff oder beide Methyl sind;
D und E ebenso entweder beide Wasserstoff oder beide Methyl repräsentieren;
C für Wasserstoff oder Methyl steht;
mit der Maßgabe, daß wenn D und E Methyl bedeuten, A und B beide Wasserstoff sind, und C für Methyl steht, aber wenn D und E Wasserstoff sind, A, B und C alle Wasserstoff oder alle Methyl sind;
X bedeutet: $—C(CH_3)_2—$, $—CH_2—$, $—CH_2CH_2—$ oder $—CH{=}CH—$;
$R_2$ ist: Wasserstoff, Halogen, SR, worin R für Wasserstoff oder Alkyl mit 1 bis 6 Kohlenstoffatomen steht, $NR^IR^{II}$, worin $R^I$ und $R^{II}$ unabhängig voneinander sind: Wasserstoff, Alkyl mit 1 bis 6 Kohlenstoffatomen, Phenyl oder Phenyl substituiert durch Alkyl mit 1 bis 6 Kohlenstoffatomen, Alkoxy mit 1 bis 6 Kohlenstoffatomen, Halogen oder Trifluormethyl;
$R_2'$ und $R_3'$ unabhängig voneinander sind: Wasserstoff, Alkanoyl mit 1 bis 6 Kohlenstoffatomen, Benzoyl oder Benzoyl substituiert durch Alkyl mit 1 bis 6 Kohlenstoffatomen, Alkoxy mit 1 bis 6 Kohlenstoffatomen, Halogen oder Trifluormethyl, oder wenn sie zusammengenommen werden, $R_2'$ und $R_3'$ Alkyliden mit 1 bis 6 Kohlenstoffatomen sein können; und
$R_6'$ ist: Halogen, Wasserstoff oder $R_5'O$, worin $R_5'$ darstellt: Wasserstoff, Alkanoyl mit 1 bis 6 Kohlenstoffatomen, Benzoyl oder Benzoyl substituiert durch Alkyl mit 1 bis 6 Kohlenstoffatomen, Alkoxy mit 1 bis 6 Kohlenstoffatomen, Halogen oder Trifluormethyl; und das NH— an das eine bzw. das andere der angrenzenden Kohlenstoffatome im Radikal IIB gebunden ist; welches Verfahren umfaßt:
die Umsetzung eines 6-Halogenpurinribosids der Formel:

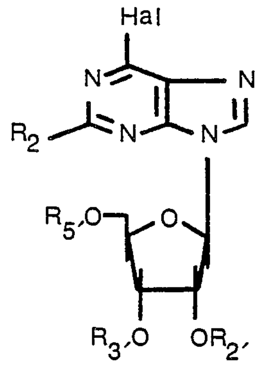

III

worin Hal Halogen bedeutet, und $R_2$, $R_2'$, $R_3'$, $R_5'$ wie oben definiert sind, mit einer Verbindung der Formel:

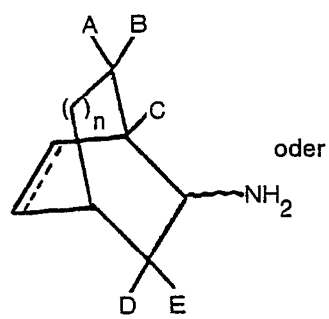

oder

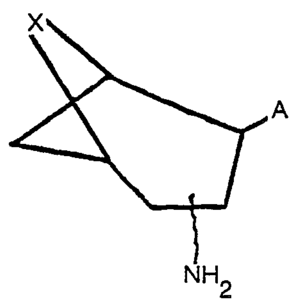

IVA IVB

in einem inerten Lösungsmittel bei etwa 25 bis etwa 130°C, von 1 bis 48 Stunden lang, und gewünschtenfalls das Überführen von $R_5'O$ in Halogen oder Methyl; und weiters, gewünschtenfalls das Überführen der resultierenden freien Base in ein pharmazeutisch akzeptables Säureadditionssalz.

2. Verfahren zur Herstellung einer wie im Anspruch 1 beanspruchten Verbindung der Formel I, worin $R_2$ für SR und $NR^IR^{II}$ steht, und $R_2'$, $R_3'$ und $R_5'$ Wasserstoff sind, welches Verfahren umfaßt:
die Umsetzung einer Verbindung der Formel

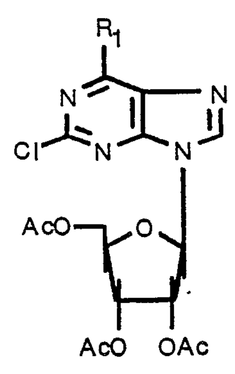

worin $R_1$ wie im Anspruch 1 definiert ist, und worin Ac für Acetyl steht, mit einem Nukleophil, welches $R_2$ entspricht; das Entfernen der Acetylgruppen mit Ammoniumhydroxid und gewünschtenfalls das Überführen der resultierenden freien Base in ein pharmazeutisch akzeptables Säureadditionssalz.

3. Verfahren zur Herstellung einer wie im Anspruch 1 beanspruchten Verbindung der Formel I, worin $R_6'$ für Chlor steht, welches Verfahren umfaßt:
die Umsetzung einer Verbindung der Formel:

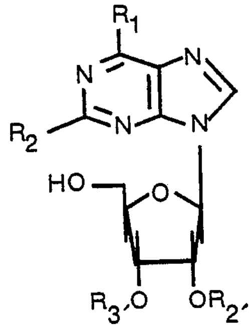

worin $R_1$ und $R_2$ wie oben definiert sind, und $R_3'$ und $R_2'$ die obenstehende Bedeutung haben, mit der zusätzlichen Maßgabe, daß keines von beiden Wasserstoff darstellt; mit Thionylchlorid und gewünschtenfalls die Bildung von sowohl $R_3'$ als auch $R_2'$ als Wasserstoff durch weitere Behandlung mit einer wässerigen Säure und gewünschtenfalls das Überführen der resultierenden freien Base in ein pharmazeutisch akzeptables Säureadditionssalz.

4. Verfahren zur Herstellung einer Verbindung der Formel I nach Anspruch 1, worin $R_6'$ für Wasserstoff steht, welches Verfahren umfaßt:
die Umsetzung einer Verbindung der Formel

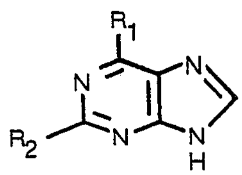

worin $R_1$ und $R_2$ wie oben definiert sind, mit einer Verbindung der Formel

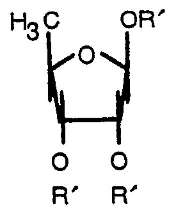

worin R' eine Schutzgruppe ist.

5. Verfahren nach irgendeinem vorhergehenden Anspruch, worin $R_2$ für Wasserstoff steht.

6. Verfahren nach irgendeinem der Ansprüche 1 bis 4, worin $R_1$ die Formel IIA hat.

7. Verfahren nach irgendeinem der Ansprüche 1 bis 4, worin $R_1$ die Formel II B hat.

8. Verfahren nach Anspruch 6, worin eine der folgenden Verbindungen hergestellt wird:
$N^6$-endo-Bicyclo[2.2.1]heptyladenosin;
$N^6$-exo-Bicyclo[2.2.1]heptyladenosin;
endo-Bicyclo[2.2.1]heptyl-5'-chloro-5'-desoxyadenosin;
$N^6$-(endo)-Norbornyl-5'-desoxyadenosin;
$N^6$-(2-endo-Norbornyl)-5'-desoxy-2',3'-diacetyladenosin;
$N^6$-Norbornyl-2',3'-O-isopropylidenadenosin; und
$N^6$-2-(endo)-Norbornyl-5'-chloro-5'-desoxy-2',3'-isopropylidenadenosin.

9. Verfahren zur Herstellung einer pharmazeutischen Zusammensetzung, welches die Kombination einer therapeutisch wirksamen Menge einer durch ein wie in irgendeinem der Ansprüche 1 bis 5 beanspruchten Verfahren hergestellten Verbindung mit einem pharmazeutisch akzeptablen Träger oder Verdünnungsmittel umfaßt.

10. Verbindung, welche mittels eines wie in irgendeinem der Ansprüche 1 bis 8 beanspruchten Verfahrens hergestellt wurde, für die Verwendung in einer Methode zur Behandlung von Schmerz bei einem daran leidenden Säuger, vorzugsweise in Dosiseinheitsform.

11. Verbindung, welche mittels eines wie in irgendeinem der Ansprüche 1 bis 8 beanspruchten Verfahrens hergestellt wurde, für die Verwendung in einer Methode zur Behandlung von Entzündung bei einem daran leidenden Säuger, vorzugsweise in Dosiseinheitsform.

**Revendications pour les Etats contractants: BE CH DE FR GB IT LI LU NL SE**

1. Composé, caractérisé par la formule (I)

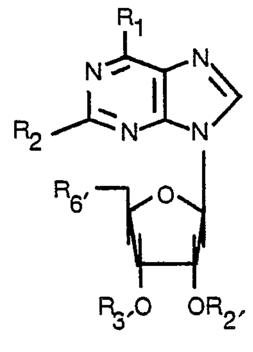

I

ses diastéréomères individuels ou leurs mélanges, ou un sel d'addition acide de celui-ci acceptable du point de vue pharmaceutique;

dans laquelle $R_1$ est de formule

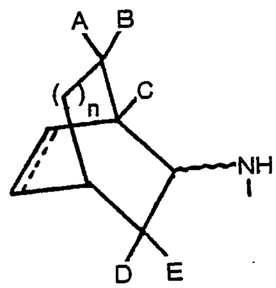

IIA

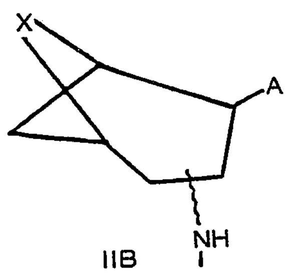

IIB

où —NH—

est soit endo, soit exo;

- - - - - est une liaison double ou simple;

n est zéro, un ou deux;

A et B sont tous les deux soit un atome d'hydrogène, soit un groupe méthyle; D et E sont aussi tous les deux soit un atome d'hydrogène, soit un groupe méthyle; C est un atome d'hydrogène ou un groupe méthyle; à la condition que lorsque D et E sont un groupe méthyle, alors A et B sont tous les deux un atome d'hydrogène et C est un groupe méthyle, mais lorsque D et E sont chacun un atome d'hydrogène, alors A, B et C sont tous un atome d'hydrogène ou tous un groupe méthyle;

X est $—C(CH_3)_2—$, $—CH_2—$, $—CH_2—CH_2—$, $—CH{=}CH—$;

$R_2$ est un atome d'hydrogène, d'halogène, SR où R est un atome d'hydrogène ou un groupe alkyle en $C_{1-6}$ $NR^{I}R^{II}$ où $R^{I}$ et $R^{II}$ sont indépendamment un atome d'hydrogène, un groupe alkyle en $C_{1-6}$, phényle ou phényle substitué par un groupe alkyle en $C_{1-6}$, alkoxy en $C_{1-6}$, un atome d'halogène ou un groupe trifluorométhyle;

$R_2'$ et $R_3'$ sont indépendamment un atome d'hydrogène, un groupe alkanoyle en $C_{1-6}$, benzoyle ou benzoyle substitué par un groupe alkyle en $C_{1-6}$, alkoxy en $C_{1-6}$, un atome d'halogène ou un groupe trifluorométhyle ou ensemble, ils peuvent former un radical alkylidène en $C_{1-6}$; et

$R_6'$ est un atome d'hydrogène, d'halogène ou bien $R_5'O$ où $R^{5'}$ est un atome d'hydrogène, un groupe alkanoyle en $C_{1-6}$, benzoyle ou benzoyle substitué par un groupe alkyle en $C_{1-6}$, alkoxy en $C_{1-6}$, un atome d'halogène ou un groupe trifluorométhyle; et le groupe NH— dans le radical IIB est fixé à l'un ou l'autre des atomes de carbone adjacents.

2. Composé suivant la revendication 1, caractérisé en ce que $R_2$ est un atome d'hydrogène.

3. Composé suivant la revendication 1, caractérisé en ce que $R_1$ est de Formule IIA.

4. Composé suivant la revendication 1, caractérisé en ce que $R_1$ est de Formule IIB.

5. Composé suivant la revendication 3, caractérisé en ce qu'il s'agit de l'un des composés suivants:

$N^6$-endo-bicyclo[2.2.1]heptyl adénosine;

$N^6$-exo-bicyclo[2.2.1]heptyl adénosine;

$N^6$-endo-bicyclo[2.2.1]heptyl-5'-chloro-5'-désoxy adénosine;

$N^6$-(endo)-norbornyl-5'-désoxyadénosine;

$N^6$-(2-endo-norbornyl)-5'-désoxy-2',3'-diacétyl adénosine;
$N^6$-norbornyl-2',3'-O-isopropylidène adénosine; et
$N^6$-2-(endo)-norbornyl-5'-chloro-5'-désoxy-2',3'-isopropylidène adénosine.

6. Procédé pour la préparation d'un composé suivant la revendication 1, caractérisé en ce qu'il comprend la réaction d'un 6-halopurine riboside de formule

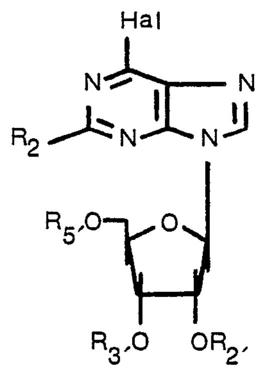

III

dans laquelle Hal est un atome d'halogène et $R_2$, $R_2'$, $R_3'$, $R_5'$ sont tels que définis dans la revendication 1, avec un composé de formule:

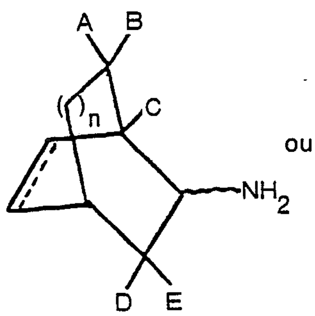

ou

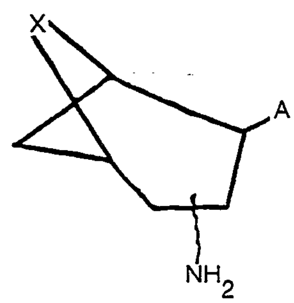

IVA IVB

dans un solvant inerte entre environ 25 et 130°C pendant de 1 à 48 heures, et si cela est désiré, la conversion de $R_5'O$ en un atome d'halogène ou un groupe méthyle; et de plus, si cela est désiré, la conversion de la base libre résultante en un sel d'addition acide acceptable du point de vue pharmaceutique.

7. Procédé pour la préparation d'un composé suivant la revendication 1 dans laquelle $R_2$ est SR ou $NR^IR^{II}$ et $R_2'$, $R_3'$ et $R_5'$ sont chacun un atome d'hydrogène, caractérisé en ce qu'il comprend la réaction d'un composé de formule

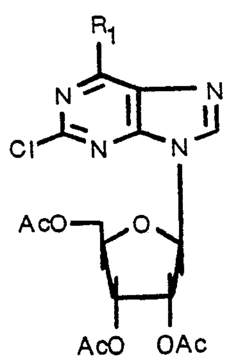

dans laquelle $R_1$ est tel que défini dans la revendication 1 et Ac est un groupe acétyle, avec un nucléophile correspondant à $R_2$; l'élimination des groupes acétyle avec de l'hydroxyde d'ammonium, et si cela est désiré, la conversion de la base libre résultant en un sel d'addition acide acceptable du point de vue pharmaceutique.

8. Procédé pour la préparation d'un composé suivant la revendication 1 dans laquelle $R_6'$ est un atome de chlore, caractérisé en ce qu'il comprend la réaction d'un composé de formule

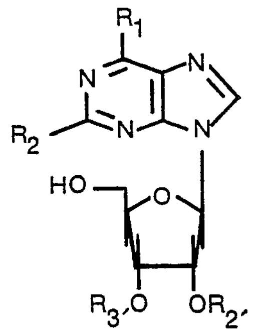

dans laquelle $R_1$ et $R_2$ sont tels que définis plus haut, $R_3'$ et $R_2'$ sont tels que définis plus haut avec la condition supplémentaire que ni l'un ni l'autre n'est un atome d'hydrogène, avec du chlorure de thionyle, et si cela est désiré, la transformation de chacun des $R_3'$ et $R_2'$ en un atome d'hydrogène par un traitement ultérieur avec un acide aqueux, et si cela est désiré, la conversion de la base libre résultante en un sel d'addition acide acceptable du point de vue pharmaceutique.

9. Procédé pour la préparation d'un composé suivant la revendication 1 dans laquelle $R_6'$ est un atome d'hydrogène, caractérisé en ce qu'il comprend la réaction d'un composé de formule

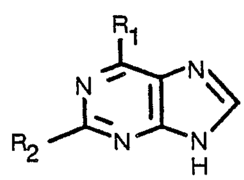

dans laquelle $R_1$ et $R_2$ sont tels que définis plus haut, avec un composé de formule

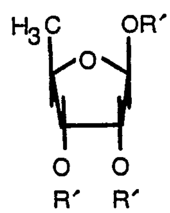

dans laquelle R' est un groupe protecteur.

10. Composition pharmaceutique, caractérisée en ce nu'elle comprend une quantité efficace du point de vue thérapeutique d'un composé suivant l'une quelconque des revendications 1 à 5 avec un support ou un diluant acceptable du point de vue pharmaceutique.

11. Utilisation dans un procédé de traitement de la douleur chez un mammifère d'un composé suivant l'une quelconque des revendications 1 à 5, de préférence sous forme de dosage unitaire.

12. Utilisation dans un procédé de traitement de l'inflammation chez un mammifère d'un composé suivant l'une quelconque des revendications 1 à 5, de préférence sous forme de dosage unitaire.

**Revendications pour l'Etat contractant: AT**

1. Procédé pour préparer un composé de formule (I)

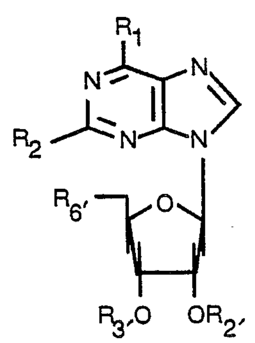

I

ses diastéréomères individuels ou leurs mélanges, ou un sel d'addition acide de celui-ci acceptable du point de vue pharmaceutique;

dans laquelle $R_1$ est de formule

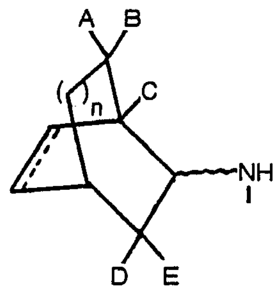

IIA

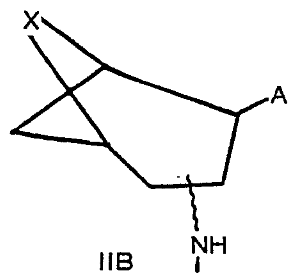

IIB

où —NH—

est soit endo, soit exo;

- - - - - est une liaison double ou simple;

n est zéro, un ou deux;

A et B sont tous les deux soit un aotme d'hydrogène, soit un groupe méthyle; D et E sont aussi tous les deux soit un atome d'hydrogène, soit un groupe méthyle; C est un atome d'hydrogène ou un groupe méthyle; à la condition que lorsque D et E sont un groupe méthyle, alors A et B sont tous les deux un atome d'hydrogène et C est un groupe méthyle, mais lorsque D et E sont chacun un atome d'hydrogène, alors A, B et C sont tous un atome d'hydrogène ou tous un groupe méthyle;

X est $—C(CH_3)_2—$, $—CH_2—$, $—CH_2—CH_2—$, $—CH{=}CH—$;

$R_2$ est un atome d'hydrogène, d'halogène, SR où R est un atome d'hydrogène ou un groupe alkyle en $C_{1-6}$ $NR^IR^{II}$ où $R^I$ et $R^{II}$ sont indépendamment un atome d'hydrogène, un groupe alkyle en $C_{1-6}$, phényle ou phényle substitué par un groupe alkyle en $C_{1-6}$, alkoxy en $C_{1-6}$, un atome d'halogène ou un groupe trifluorométhyle;

$R_2'$ et $R_3'$ sont indépendamment un atome d'hydrogène, un groupe alkanoyle en $C_{1-6}$, benzoyle ou benzoyle substitué par un groupe alkyle en $C_{1-6}$, alkoxy en $C_{1-6}$, un atome d'halogène ou un groupe trifluorométhyle ou ensemble, ils peuvent former un radical alkylidène en $C_{1-6}$; et

$R_6'$ est un atome d'hydrogène, d'halogène ou bien $R_5'O$ où $R^{5'}$ est un atome d'hydrogène, un groupe alkanoyle en $C_{1-6}$, benzoyle ou benzoyle substitué par un groupe alkyle en $C_{1-6}$, alkoxy en $C_{1-6}$, un atome d'halogène ou un groupe trifluorométhyle; et le groupe NH— dans le radical IIB est fixé à l'un ou l'autre des atomes de carbone adjacents.

caractérisé en ce qu'il comprend la réaction d'un 6-halopurine riboside de formule

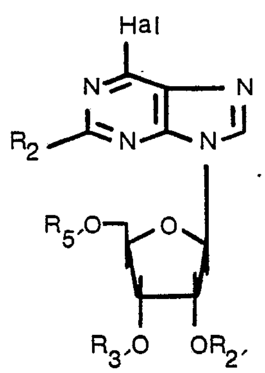

III

dans laquelle Hal est un atome d'halogène et $R_2$, $R_2'$, $R_3'$, $R_5'$ sont tels que définis plus haut, avec un composé de formule:

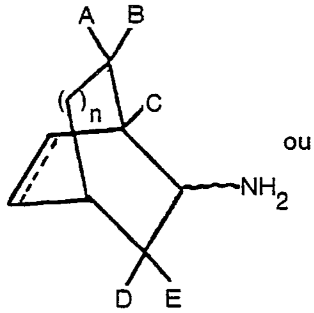

ou

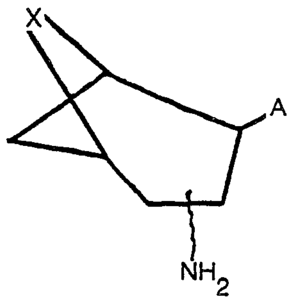

IVA IVB

dans un solvant inerte entre environ 25 et 130°C pendant de 1 à 48 heures, et si cela est désiré, la conversion de $R_5$'O en un atome d'halogène ou un groupe méthyle; et de plus, si cela est désiré, la conversion de la base libre résultante en un sel d'addition acide acceptable du point de vue pharmaceutique.

2. Procédé pour la préparation d'un composé de formule 1 suivant la revendication 1 dans laquelle $R_2$ est SR ou $NR^iR^{ii}$ et $R_2'$, $R_3'$ et $R_5'$ sont chacun un atome d'hydrogène, caractérisé en ce qu'il comprend la réaction d'un composé de formule

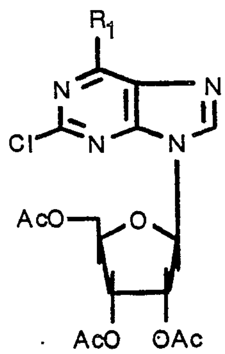

dans laquelle $R_1$ est tel que défini dans la revendication 1 et Ac est un groupe acétyle, avec un nucléophile correspondant à $R_2$; l'élimination des groupes acétyle avec de l'hydroxyde d'ammonium, et si cela est désiré, la conversion de la base libre résultant en un sel d'addition acide acceptable du point de vue pharmaceutique.

3. Procédé pour la préparation d'un composé de formule I suivant la revendication 1 dans laquelle $R_6'$ est un atome de chlore, caractérisé en ce qu'il comprend la réaction d'un composé de formule

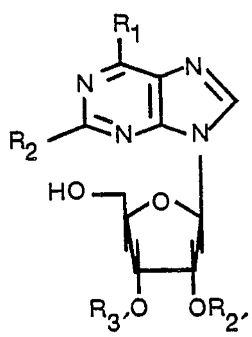

dans laquelle $R_1$ et $R_2$ sont tels que définis plus haut, $R_3'$ et $R_2'$ sont tels que définis plus haut avec la condition supplémentaire que ni l'un ni l'autre n'est un atome d'hydrogène, avec du chlorure de thionyle, et si cela est désiré, la transformation de chacun des $R_3'$ et $R_2'$ en un atome d'hydrogène par un traitement ultérieur avec un acide aqueux, et si cela est désiré, la conversion de la base libre résultante en un sel d'addition acide acceptable du point de vue pharmaceutique.

4. Procédé pour la préparation d'un composé de formule I suivant la revendication 1 dans laquelle $R_6'$ est un atome d'hydrogène, caractérisé en ce qu'il comprend la réaction d'un composé de formule

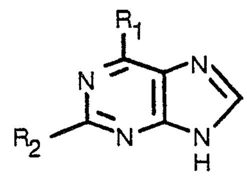

dans laquelle $R_1$ et $R_2$ sont tels que définis plus haut, avec un composé de formule

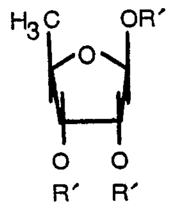

dans laquelle R′ est un groupe protecteur.

5. Procédé suivant l'une quelconque des revendications précédentes, caractérisé en ce que $R_2$ est un atome d'hydrogène.

6. Procédé suivant l'une quelconque des revendications 1 à 4, caractérisé en ce que $R_1$ est de Formule IIA.

7. Procédé suivant l'une quelconque des revendications 1 à 4, caractérisé en ce que $R_1$ est de Formule IIB.

8. Procédé suivant la revendication 6, caractérisé en ce que l'on prépare l'un des composés suivants:
N[6]-endo-bicyclo[2.2.1]heptyl adénosine;
N[6]-exo-bicyclo[2.2.1]heptyl adénosine;
N[6]-endo-bicyclo[2.2.1]heptyl-5′-chloro-5′-désoxy adénosine;
N[6]-(endo)-norbornyl-5′-désoxyadénosine;
N[6]-(2-endo-norbornyl)-5′-désoxy-2′,3′-diacétyl adénosine;
N[6]-norbornyl-2′,3′-O-isopropylidène adénosine; et
N[6]-2-(endo)-norbornyl-5′-chloro-5′-désoxy-2′,3′-isopropylidène adénosine.

9. Procédé pour la préparation d'une composition pharmaceutique, caractérisé en ce qu'il comprend la combinaison d'une quantité efficace du point de vue thérapeutique d'un composé préparé suivant le procédé de l'une quelconque des revendications 1 à 5 avec un support ou un diluant acceptable du point de vue pharmaceutique.

10. Utilisation dans un procédé de traitement de la douleur chez un mammifère d'un composé préparé suivant le procédé de l'une quelconque des revendications 1 à 8, de préférence sous forme de dosage unitaire.

11. Utilisation dans un procédé de traitement de l'inflammation chez un mammifère d'un composé préparé suivant le procédé de l'une quelconque des revendications 1 à 8, de préférence sous forme de dosage unitaire.